## Europäisches Patentamt
## European Patent Office
## Office européen des brevets

(19)

(11) Veröffentlichungsnummer : **0 345 688 B1**

(12)

# EUROPÄISCHE PATENTSCHRIFT

(45) Veröffentlichungstag der Patentschrift :
**11.11.92 Patentblatt 92/46**

(51) Int. Cl.$^5$ : **C07C 211/45, C07C 209/10**

(21) Anmeldenummer : **89110129.7**

(22) Anmeldetag : **05.06.89**

(54) **Verfahren zur Herstellung von 4-Nitro-3-trifluormethyl-anilin.**

(30) Priorität : **09.06.88 DE 3819565**

(43) Veröffentlichungstag der Anmeldung :
**13.12.89 Patentblatt 89/50**

(45) Bekanntmachung des Hinweises auf die
Patenterteilung :
**11.11.92 Patentblatt 92/46**

(84) Benannte Vertragsstaaten :
**BE CH DE FR GB IT LI NL**

(56) Entgegenhaltungen :
**EP-A- 0 166 533**
**US-A- 4 302 599**

(56) Entgegenhaltungen :
**HOUBEN WEYL** "Methoden der organischen
**Chemie"**, 4. Auflage, Band XI/1
"**Stickstoffverbindungen II**", 1957, **GEORG
THIEME VERLAG,Stuttgart, Seiten 63-68**

(73) Patentinhaber : **HOECHST
AKTIENGESELLSCHAFT
Postfach 80 03 20
W-6230 Frankfurt am Main 80 (DE)**

(72) Erfinder : **Papenfuhs, Theodor, Dr.
Heinrich-Bleicher-Strasse 40
W-6000 Frankfurt am Main 50 (DE)**
Erfinder : **Hess, Reiner, Dr.
Lärchenstrasse 4
W-6200 Wiesbaden (DE)**

EP 0 345 688 B1

## Beschreibung

4-Nitro-3-trifluormethyl-anilin dient als wertvolles Vorprodukt bei der Synthese von Wirkstoffen für Hautcremes (US 4 299 845), für die Empfängnisverhütung (EP 2892; CH 572 019) und für Pflanzenwuchsregulatoren (FR 2 374 847).

Als technische Synthese ist in US 4 302 599 folgende Arbeitsweise beschrieben:
Man acyliert in erster Stufe 3-Trifluormethyl-anilin mit Isobutyrylchlorid zu 3-Trifluormethyl-isobutyranilid. Dieses wird in der zweiten Verfahrensstufe zunächst in 15 - 18 % Oleum gelöst und dann mit 90 %iger Salpetersäure bei 5°C nitriert. Die Aufarbeitung erfolgt durch Verdünnen mit der fünffachen Ansatzmenge an Wasser. In der dritten Verfahrensstufe wird das entstandene 4-Nitro-3-trifluormethylisobutyranilid alkalisch oder sauer in Wasser, einem wassermischbaren Lösungsmittel und einem Alkali- oder Erdalkalicarbonat oder -hydroxid verseift, wobei bevorzugt im sauren Medium - mit starken Mineralsäuren in Gegenwart starker organischer Säuren, beispielsweise Alkyl- oder Aryl-Sulfonsäuren - gearbeitet wird. In der genannten Patentschrift sind für keine Verfahrensstufen die Ausbeuten und Schmelzpunkte angegeben, weshalb man relativ unbefriedigende Umsetzungen unterstellen kann. Hinzu kommen die zahlreichen Verfahrensstufen und der damit verbundene Chemikalienaufwand, welche die technische Realisierung der beschriebenen Verfahrensweise aus verfahrenstechnischer und ökologischer Sicht problematisch machen:

Die Nitrierung in Oleum mit nachfolgender starker Verdünnung schafft eine nur schwer regenerierbare, weil zu wenig konzentrierte, organisch belastete Abfallsäure.

Die hydrolytische Abspaltung der Schutzgruppe in Gegenwart starker Säuren unter Bildung von Isobuttersäure (Geruchsbelästigung) kann nur mit einem großen Überschuß der Säure erfolgen, aus der das gewünschte 4-Nitro-3-trifluormethyl-anilin durch Filtration isoliert wird. Neben dem zur Abtrennung aus starken Säuren erforderlichen hohen apparativen Aufwand resultiert dabei ein organisch hochbelastetes saures Filtrat, da zum einen die gebildete Isobuttersäure im Filtrat gelöst bleibt, zum anderen als Zusätze organische Sulfonsäuren im Hydrolysemedium enthalten sind. Dessen Aufarbeitung ist nur durch Verbrennung in einer säurefesten Anlage möglich, da es biologisch aufgrund der hohen organischen und anorganischen Last nicht abbaubar ist.

Es bestand daher ein Bedarf an einem Verfahren zur Herstellung von 4-Nitro-3-trifluormethyl-anilin, das die aufgezeigten Nachteile weitgehend vermeidet und technisch und ökologisch bei Erzielung hoher Ausbeuten vorteilhaft durchführbar ist.

Es wurde nun gefunden, daß man 4-Nitro-3-trifluormethyl-anilin in nahezu quantitativer Ausbeute herstellen kann, indem man 1 Mol 5-Chlor-2-nitrobenzotrifluorid mit mindestens 10 Mol wäßrigen, 20 bis etwa 60 %igen, vorzugsweise etwa 24 bis etwa 40 %igen Ammoniaks bei Temperaturen von etwa 170 bis etwa 230°C, vorzugsweise etwa 180 bis etwa 200°C, bei einem Druck bis zu etwa 40 bar, gegebenenfalls in Gegenwart katalytischer Mengen Kupfer oder Kupferverbindungen, umsetzt.

Zwar kann man auch bei Temperaturen unterhalb 170°C bzw. oberhalb 230°C eine Umsetzung herbeiführen, doch verläuft die Reaktion unterhalb von 170°C nicht mit ausreichender Geschwindigkeit und Temperaturen oberhalb 230°C sind wegen der dabei auftretenden Drücke und beginnender Verharzung weniger vorteilhaft.

Es ist zweckmäßig, das Ammoniak in einem molaren Überschuß von 10 bis etwa 40, vorzugsweise etwa 10 bis etwa 15, bezogen auf das 5-Chlor-2-nitrobenzotrifluorid, anzuwenden. Nichtumgesetztes Ammoniak kann in den Prozeß zurückgeführt werden. Bei der Umsetzung entsteht als Nebenprodukt lediglich 1 Mol Ammoniumchlorid.

An katalytisch wirksamen Kupferverbindungen seien beispielsweise genannt Kupfer(II)-carbonat, Kupfer(I)-chlorid und Kupfer(I)-oxid.

Die Umsetzung kann diskontinuierlich oder kontinuierlich durchgeführt werden. Wegen der auftretenden Drücke von etwa 30 bis 40 bar kann eine kontinuierliche Umsetzung, beispielsweise in einer von außen beheizten Reaktionsschlange, infolge des geringen Raum- und damit Apparatebedarfs von Vorteil sein.

Bekanntlich kann man bei Standardbedingungen durch Einleiten von Ammoniak in Wasser nur eine etwa 25 %ige Lösung erzielen. Will man höher konzentrierte wäßrige Ammoniaklösungen herstellen, so muß das Wasser bzw. die wäßrige Ammoniaklösung abgekühlt werden oder man leitet flüssiges Ammoniak, aus einem Druckgefäß in eine wäßrige Ammoniaklösung ein (vgl. hierzu das nachstehende Beispiel 2).

Das als Ausgangsprodukt benötigte 5-Chlor-2-nitro-benzotrifluorid ist durch kontinuierliche Nitrierung von 3-Chlorbenzotrifluorid zugänglich. Das entstandene Isomerengemisch enthält etwa 90 % des gewünschten 5-Chlor-2-nitro-benzotrifluorids und wird durch Schmelzkristallisation gereinigt. Man kommt mit dieser Verfahrensweise auf ein 5-Chlor-2-nitrobenzotrifluorid eines Reingehaltes von > 98 %, welches direkt in die erfindungsgemäße Ammonolyse eingesetzt werden kann.

Nach dem erfindungsgemäßen Verfahren kann das 4-Nitro-3-trifluormethyl-anilin somit wesentlich einfacher als nach dem bisher bekannten Verfahren hergestellt werden. Die Qualität des Produktes erlaubt seinen Einsatz bei der Herstellung von Wirkstoffen ohne weitere Reinigung.

Aus HOUBEN-WEYL, Band XI/1 (1957), Seiten 63 - 64 ist die Umsetzung von Nitro-chlorbenzolen mit wäßrigem Ammoniak einer Konzentration von 27 % und mehr prinzipiell bekannt. Aus Chemical Abstracts, Band 94, Nr. 9 (1981), Seite 646, Abstract Nr. 64770z ist ferner bekannt, daß die Umsetzung von o-Nitro-chlorbenzol mit Ammoniak durch Kupfer oder Kupferverbindungen katalysiert wird. Aus der erstgenannten Literaturstelle ist aber auch bekannt, daß ein zur Nitrogruppe o-ständiges Chloratom erheblich leichter nucleophil substituiert werden kann als ein p-ständiges. So kann das im 2,5-Dichlor-nitrobenzol 2-ständige Chloratom durch eine Aminogruppe mittels wäßrigem Ammoniak ausgetauscht werden, wobei das 2-Nitro-4-chlor-anilin in 99 %iger Ausbeute erhalten wird (siehe Tabelle 10, Seite 64, Beispiel 4 der genannten Literaturstelle von HOUBEN-WEYL). Dagegen ist aus den Beispielen 1 und 2 der genannten Literaturstelle von HOUBEN-WEYL ersichtlich, daß die Umsetzung von 4-Nitrochlorbenzol mit Ammoniak unter sehr drastischen, sicherheitstechnisch nicht mehr vertretbaren Bedingungen möglich ist.

Im Hinblick darauf, daß beim erfindungsgemäßen Verfahren die Nitrogruppe und das Chloratom in 1,4-Stellung zueinander stehen, das am aromatischen Kern eine den Chloraustausch nicht beeinflussende Trifluormethyl-Gruppe (in 3-Stellung zum Chloratom) enthält, war zu erwarten, daß der Chloraustausch entweder nur unter den in den Beispielen 1 und 2 der weiter oben genannten Literaturstelle von HOUBEN-WEYL beschriebenen drastischen Bedingungen oder überhaupt nicht stattfindet. Umso überraschender war es daher, festzustellen, daß die Umsetzung von 5-Chlor-2-nitro-benzotrifluorid - sowohl in Gegenwart als auch in Abwesenheit von Kupfer oder Kupferverbindungen - mit höher bis hochprozentigem wäßrigem Ammoniak in selektivem Chloraustausch in hohen Ausbeuten und auf ökologisch vorteilhafte Weise zum 4-Nitro-3-trifluormethyl-anilin führt.

Die nachstehenden Beispiele dienen zur Erläuterung der Erfindung, ohne sie darauf zu beschränken. Die Prozentangaben beziehen sich auf das Gewicht, sofern nichts anderes angegeben ist.

**Beispiel 1**

In einem 5-1-Stahlautoklav wurde ein Gemisch aus 452 g 5-Chlor-2-nitro-benzotrifluorid und 2125 g 24 %iger wäßriger Ammoniaklösung unter gutem Rühren für 7 Stunden auf 175°C erhitzt. Dabei baute sich ein Druck von 32 bar auf. Danach wurde auf Raumtemperatur abgekühlt, überschüssiges Ammoniak abgeblasen und die Reaktionslösung über eine Nutsche abgesaugt. Als Rückstand wurden 391 g feuchtes, kristallines 4-Nitro-3-trifluomethyl-anilin erhalten, welches nach Trocknung im Vakuumtrockenschrank 375 g trockenes 4-Nitro-3-trifluormethyl-anilin, entsprechend 91 % der Theorie (Fp.: 126-127°C) ergab. Nach spektroskopischen, gas- und hochleistungsflüssig-chromatographischen Untersuchungen enthielt das Reaktionsprodukt kein 5-Chlor-2-nitro-benzotrifluorid, aber mehr als 98 % 4-Nitro-3-trifluormethyl-anilin.

**Beispiel 2**

In einem 5-1-Stahlautoklav wurden 904 g 5-Chlor-2-nitro-benzotrifluorid, 2833 g 24 %ige wäßrige Ammoniaklösung und 170 g flüssiges Ammoniak unter Rühren 8 Stunden auf 175°C erhitzt. Dabei baute sich ein Druck von 36 bar auf. Danach wurde auf Raumtemperatur abgekühlt, überschüssiges Ammoniak abgeblasen und die Rohlösung über eine Nutsche abgesaugt. Erhalten wurden 904 g feuchtes 4-Nitro-3-trifluormethyl-anilin, welches nach Trocknung bei 50°C im Vakuumtrockenschrank 776,2 g trockenes 4-Nitro-3-trifluormethyl-anilin, entsprechend 94,2 % der Theorie (Fp.: 127-128°C) ergab, dessen chromatographische und spektroskopische Eigenschaften sich mit dem gemäß Beispiel 1 erhaltenen Produkt decken.

Verfährt man wie vorstehend beschrieben, lediglich zusätzlich in Gegenwart katalytischer Mengen an Kupfer(II)-carbonat, so erzielt man das Ergebnis des Beispiels 2 bei schnellerer Umsetzung.

**Beispiel 3** (Vergleichsbeispiel)

In einem 2-1-Stahlautoklav wurde ein Gemisch aus 226 g 5-Chlor-2-nitro-benzotrifluorid, 560 g 22 %igem wäßrigen Ammoniak und 300 g Isopropanol unter gutem Rühren 5 Stunden auf 180°C erwärmt, wobei sich ein Druck von 28 bar aufbaute. Danach wurde auf Raumtemperatur kaltgerührt, das überschüssige Ammoniak abgeblasen und die Rohlösung, nach Verdünnen mit weiteren 300 g Isopropanol, von gebildetem Ammoniumchlorid abgesaugt. Nach Zugabe von 1000 ml Wasser wurden 630 g Isopropanol-Wasser-Azeotrop abdestilliert. Anschließend wurde auf Raumtemperatur kaltgerührt und die wäßrige Suspension von 4-Nitro-3-trifluormethyl-anilin abgesaugt. Man erhielt nach Trocknung im Vakuumtrockenschrank eine Ausbeute von 126 g 4-Nitro-3-trifluormethyl-anilin, entsprechend einer Ausbeute von 62 % der Theorie (Fp.: 110-116°C).

**Patentansprüche**

1. Verfahren zur Herstellung von 4-Nitro-3-trifluormethyl-anilin, dadurch gekennzeichnet, daß man 5-Chlor-2-nitrobenzotrifluorid mit mindestens der 10fachen molaren Menge wäßrigen Ammoniak einer Konzentration von 20 bis etwa 60 Gewichtsprozent bei Temperaturen von etwa 170 bis etwa 230°C bei einem Druck bis zu etwa 40 bar umsetzt.

2. Verfahren nach Anspruch 1, dadurch gekennzeichnet, daß man bei etwa 180 bis etwa 200°C umsetzt.

3. Verfahren nach mindestens einem der Ansprüche 1 und 2, dadurch gekennzeichnet, daß man mit wäßrigem Ammoniak einer Konzentration von etwa 24 bis etwa 40 Gewichtsprozent umsetzt.

4. Verfahren nach mindestens einem der Ansprüche 1 bis 3, dadurch gekennzeichnet, daß man in Gegenwart katalytischer Mengen Kupfer oder Kupferverbindungen umsetzt.

5. Verfahren nach mindestens einem der Ansprüche 1 bis 4, dadurch gekennzeichnet, daß man in Gegenwart von Kupfer(II)-carbonat, Kupfer(I)-chlorid oder Kupfer(I)-oxid umsetzt.

6. Verfahren nach mindestens einem der Ansprüche 1 bis 5, dadurch gekennzeichnet, daß man die Umsetzung kontinuierlich vornimmt.

**Claims**

1. A process for the preparation of 4-nitro-3-trifluoromethyl-aniline, which comprises reacting 5-chloro-2-nitro-benzotrifluoride with at least 10 times the molar amount of aqueous ammonia having a concentration of 20 to about 60 percent by weight at temperatures of about 170 to about 230°C under a pressure of up to about 40 bar.

2. The process as claimed in claim 1, wherein the reaction is carried out at about 180 to about 200°C.

3. The process as claimed in at least one of claims 1 and 2, wherein the reaction is carried out with aqueous ammonia having a concentration of about 24 to about 40 percent by weight.

4. The process as claimed in at least one of claims 1 to 3, wherein the reaction is carried out in the presence of catalytic amounts of copper or copper compounds.

5. The process as claimed in at least one of claims 1 to 4, wherein the reaction is carried out in the presence of copper(II) carbonate, copper(I) chloride or copper(I) oxide.

6. The process as claimed in at least one of claims 1 to 5, wherein the reaction is carried out continuously.

**Revendications**

1. Procédé pour préparer la 4-nitro-3-trifluorométhyl-aniline, procédé caractérisé en ce qu'on fait réagir le 1-trifluorométhyl-2-nitro-5-chloro-benzène avec au moins dix fois sa quantité molaire d'ammoniac sous la forme d'une solution aqueuse d'une concentration comprise entre 20 et environ 60 % en poids, à des températures d'environ 170 à environ 230 °C et sous une pression pouvant aller jusqu'à environ 40 bar.

2. Procédé selon la revendication 1 caractérisé en ce qu'on effectue la réaction à une température comprise entre environ 180 et environ 200 °C.

3. Procédé selon au moins une des revendications 1 et 2, caractérisé en ce qu'on effectue la réaction avec une solution ammoniacale aqueuse d'une concentration comprise entre environ 24 et environ 40 % en poids.

4. Procédé selon au moins une des revendications 1 à 3, caractérisé en ce qu'on effectue la réaction en présence de quantités catalytiques de cuivre ou de composés du cuivre.

5. Procédé selon au moins une des revendications 1 à 4, caractérisé en ce qu'on effectue la réaction en présence de carbonate de cuivre(II), de chlorure de cuivre(I) ou d'oxyde de cuivre(I).

6. Procédé selon au moins une des revendications 1 à 5, caractérisé en ce qu'on effectue la réaction en continu.